# EUROPEAN PATENT SPECIFICATION

(11) **EP 1 936 375 B1**
(45) Date of publication and mention of the grant of the patent: **31.03.2010**
(21) Application number: 08005522.1
(22) Date of filing: 19.01.2001
(51) Int. Cl.: G01N 33/49, G01N 33/72, G01N 33/52, G01N 27/26, G01N 33/53

(54) **Non-enzymatic disposable electrode strip comprising a surfactant for detecting uric acid; method for producing the same and its use**
Nicht enzymatischer Einwegerkennungselektrodenstreifen der einen oberflächenaktiven Stoff beinhaltet, zum Nachweis von Harnsäure; Herstellungsverfahren dafür und Verwendung davon
Bande d'électrode jetable à détection non enzymatique comprennante un agent tensioactif pour détecter l'acide urique; son procédé de production et son utilisation

(43) Date of publication of application: 25.06.2008
(62) Divisional of application: 01300476.7
(73) Proprietor: Apex Biotechnology Corporation, Hsinchu Science Park Hsinchu (TW)
(72) Inventor: Lin, Yueh-Hui, Hsinchu (TW); Shen, Thomas Y. S., Hsinchu (TW)
(74) Representative: Sexton, Jane Helen

(56) References cited:
- EP-A- 1 025 758
- US-A- 4 301 412
- US-A- 4 713 165
- US-A- 4 876 205
- US-A- 5 556 533
- US-A- 5 779 867
- US-A- 6 054 039
- DATABASE WPI Section Ch, Week 200035 Derwent Publications Ltd., London, GB; Class B04, AN 2000-409729 XP002175754 & TW 369 411 A (WU DING BIOTECHNOLOGY CO LTD) 11 September 1999 (1999-09-11) & US 6 258 223 B1 (APEX BIOTECHNOLOGY CORP.) 10 July 2001 (2001-07-10)
- DATABASE WPI Section Ch, Week 200132 Derwent Publications Ltd., London, GB; Class B04, AN 2001-307008 XP002175755 & TW 416 005 A (APEX BIOTECHNOLOGY CORP) 21 December 2000 (2000-12-21) & DE 100 18 959 A (APEX BIOTECHNOLOGY CORP) 28 June 2001 (2001-06-28)

## Description

The present invention relates to a disposable electrode strip for detecting biomolecules, particularly uric acid.

The detection of biomolecules can be carried out by two types of electrodes: enzyme electrode and non-enzyme electrode. For clinical biochemical analysis, biomolecules are detected through a reduction or an enzymatic method. Enzyme electrodes cannot be mass-produced because of the high costs caused by strict preservation conditions, complicated manufacturing procedures and control conditions. Enzyme electrodes are not disposable and suitable for household use. On the contrary, non-enzyme electrodes can be made of disposable material and are easy to produce, store and use; therefore, they are advantageous bio-analyzers for detecting biomolecules such as uric acid and hemoglobin.

Up to now, there are no effective disposable electrode strip for the detection of the uric acid and hemoglobin. Ai-min Yu, Hai-li Zhang and Hong-yuan Chen (Analyst, 1997; 122:839~841) described the improved voltammeric behavior of uric acid and its trace determination at a polyglycine chemically modified electrode. The electrode is merely tested in the sample of uric acid in a 0.1 M phosphate buffer rather than the whole-blood sample. Such electrode has the disadvantages as follows: (1) the linear ranges are out of normal diagnostic ranges; (2) such electrode is not suitable for detecting blood sample; (3) the electrode manufacturing process is time-consuming and complicated and not suitable for mass production; and (4) the electrode costs high, and thus can not be used as a disposable strip.

Jyh-Myng Zen and Jen-Sen Tang (Anal. Chem. 1995; 67:1892-1895) modified the glassy carbon electrode by Nafion/Ru₂₋ₓPbₓO₇₋ₓ (ruthenium oxide pyrochlore) and detected uric acid by Osteryoung square-wave voltammeter. The detection of the electrode is suitable in an acidic uric acid solution with pH 1. The electrode is not suitable for detecting blood sample. Moreover, such electrode has the disadvantages as follows: (1) the price of the electrode is expensive; (2) the detectable ranges do not meet the diagnostic detection ranges; (3) the electrode manufacturing process is complex and not suitable for mass production; and (4) the electrode can not be used as a disposable strip and thus is not suitable for household use.

As to the detection of the hemoglobin, the Cyanmet-hemoglobin method is commonly used in the art. Said method uses Drabkin's solution to solve hemoglobin, i.e. an oxidization of the Fe²⁺ of hemoglobin to Fe³⁺ with K₃Fe(CN)₆ to form methemoglobin (MHb). Further, said MHb combines with KCN to form a stabilized cyanmet-hemoglobin. The concentration of hemoglobin can be obtained by measuring absorption at 540nm using optical colorimetry. Based on the oxidization of Fe²⁺ of hemoglobin with K₃Fe(CN)₆, the hemoglobin can be detected through a suitable electrode and a detectable oxidation potential. However, the above-mentioned method has a disadvantage of causing pollution of KCN having a high toxicity.

TW 369411-A describes a disposable electrode test block for current-type non-enzymatic uric acid test.

US 5556533-A describes a voltage applying method for hydrogen-type enzyme electrode.

Given the above, there is a need to develop a disposable, easy to use and inexpensive electrode strip in the detection of biomolecules, in particular the uric acid and hemoglobin.

### SUMMARY OF THE INVENTION

One object of the invention is to provide a non-enzymatic disposable uric acid detecting electrode strip, comprising an electric insulating substrate, a conducting film, an electric insulating film and a reaction film.

Another object of the invention is to provide a method for producing a disposable non-enzymatic uric acid detecting electrode strip.

Another object of the invention is to provide a uric acid detecting equipment.

Another further object of the invention is to provide a method for detecting the concentration of uric acid in a liquid sample.

### BRIEF DESCRIPTION OF THE DRAWINGS

FIG. 1a is a top view diagram of an electrode strip of the present invention.
FIG.lb is a front view diagram of an electrode strip of the present mvention.
FIG.2a shows the step of coating the conducting film 2 on electric insulating substrate 1 to form at least an anode and a cathode.
FIG.2b shows the step of partially covering an electric insulating film on the conducting film. The uncovered conducting film contains an anode connector 6, a cathode connector 7, a working electrode 8 and a reference electrode 9.
FIG.2c shows the step of coating a reaction film 4 on a reaction region formed by coating working electrode and the reference electrode.
FIG.2d shows the step of coating a reaction film with a protection film 5.
FIG.3 shows the concentration of uric acid in whole-blood respectively detected by the electrode strip of the present invention and uricase method in combination with EPAC bio-analyzer.

### BRIEF DESCRIPTION OF THE ELEMENTS NUMERALS

- 1: Insulating substrate
- 2: Conducting film
- 3: Insulating film
- 4: Reaction film
- 5: Protection film
- 6: Anode connector
- 7: Cathode connector
- 8: Working electrode
- 9: Reference electrode

### DETAILED DESCRIPTION OF THE INVENTION

The present invention features a disposable detecting electrode strip modified by a water-soluble redox electron mediator and a surfactant. The electrode strip detects the concentration of biomolecules in a liquid sample by catching signals generated by a redox current of a redox electron mediator and the biomolecules such as uric acid

It is surprisingly found that the electrode modified by a low concentration of cationic surfactant has a high reaction velocity and accuracy in the detection of uric acid.

### Electrode Article

One object of the invention is to provide a disposable electrode article eg. in the form of a strip which is suitable for the non-enzymatic detection of uric acid, which article comprises:
(1) an electric insulating substrate;
(2) a conducting film coated on one side of said insulating substrate to form an isolated and disconnected anode, and an isolated and disconnected cathode;
(3) an electric insulating film coated on a part of said conducting film,
   wherein one end of an uncovered anode of the conducting film forms at least a reference electrode and the other end an anode connector, and one end of an uncovered cathode of the conducting film forms at least a working electrode and the other end a cathode connector; and
(4) a reaction film comprising a carrier, a conductive mediator and a cationic surfactant with the concentration 0.005% to 0.05%
and being coated on a region containing at least said working electrode and said reference electrode so as to connect said working electrode and said reference electrode individually.

According to the invention, the electric insulating substrate has a flat surface and an insulation property. Said substrate is thermal-resistant more than 40°C, preferably 40-80°C, which can increase the conductivity through thermal processing. Any materials with the above-mentioned properties are suitable for the electric insulating substrate of the invention. Preferably, such materials are selected from the group consisting of polyvinyl chloride (PVC), fiber glass (FR-4), polyester suphone, bakelite plate, polyethylene terephthalate (PET) plate, polycarbonate (PC) plate, glass plate and ceramics plate (CEM-1).

According to the invention, the conducting film is coated on one side of said insulting substrate to form an isolated and disconnected cathode and an isolated and disconnected anode. Preferably, the coating is achieved by screen printing with printing ink or sticking with a metal film. More preferably, the metal film is selected from the group consisting of gold, silver, platinum and padium. The printing ink is selected from the group consisting of carbon ink, gold ink, silver ink, the mixture of carbon and silver ink, volatile graphite, copper ink, or the mixture of the above (for example, printing silver ink first and then printing carbon ink). Said cathode is partially covered by said electric insulating film and the uncovered ends of the cathode form a reference electrode and a cathode connector, respectively. The anode is also partially covered by said electric insulating film and the uncovered ends of the anode form a working electrode and anode connector respectively. The reference electrode of the cathode is covered by the reaction film and cooperates together with the working electrode of the anode to detect the induced electric effect. The cathode and anode connectors are used to connect with the amperometric sensor.

According to the invention, the electric insulating film is coated on one surface of said electric insulating substrate but does not insulate the above-mentioned cathode connector, anode connector, working electrode and reference electrode. The region uncovered by the electric insulating film including the working electrode and the reference electrode forms a reaction region, which is then coated by the reaction film for testing samples. Typically the thickness of the electric insulating films is 0.1mm to 2mm, preferably more than 0.6 mm.

According to the invention, the reaction film is coated on the reaction region by any suitable method, preferably by pipetting or screen printing. The reaction film consists of a water-soluble redox electron mediator, a carrier and a surfactant. The reaction film contacts with a sample and reacts electrochemically. Further, the reaction film can be covered with a protection film.

The conductive mediator is a material for donating or accepting electrons, that can be used as the mediator for transferring electron between the tested analyte and electrode in the redox reaction. According to the invention, said conductive mediator comprises an electrolyte with a lower redox potential than that of the tested samples. The conductive mediator will change from oxidation state to reduction state after reacting with tested sample. The conductive mediator can then reverse to oxidation state by applying a forced voltage. The changes of, such as potential, resistance, or current caused by electrochemical reaction, can be transferred through the conducting film, i.e. from the working electrode and the reference electrode connected with the reaction film to the cathode connector and the anode connector. Typically therefore a conductive mediator suitable for 100mV to 500mV of redox potential difference can be used in the invention.

Preferably, said conductive mediator is selected from the group consisting of potassium ferricyanide, ferricinium, tetrathisfulvalene, methylviologen and ferricyanide. More preferably, said conductive mediator is potassium ferricyanide. The amount of said conductive mediator preferably ranges from 0.3 to 5% by weight of the reaction film.

According to the invention, the carrier absorbs a sample because the sample is hydrophilic and hard to attach to a hydrophobic conducting film. The carrier can enhance the absorption of sample, and enhance the signals to be transferred to the conducting film. Said reaction film contacts with a sample and reacts electrochemically with said sample. Therefore, the carrier can be distributed all over the reaction film area and the signals be thoroughly transferred. Preferably, the carrier is selected from the group consisting of microcrystalline cellulose, carboxymethyl cellulose or methyl cellulose. More preferably, the celluloses have a size of below 100 µm. Preferably, the amount of the celluloses ranges from about 0.01% to about 0.1 % by weight of the reaction film.

According to the invention, the carrier optionally comprises a polymer. The polymer is to facilitate the reaction film having a certain viscosity. The reaction film can therefore be distributed thoroughly. Preferably, the polymer is selected from the group consisting of polyvinyl alcohol (PVA), polyvinyl pyrrolidone (PVP), polyethylene glycol (PEG), gelatin and the mixture thereof. More preferably, the amount of the polymer ranges from 0 to 15% by weight of the reaction film.

According to the invention, for the detection of uric acid, the surfactant used in said reaction film is a cationic surfactant with the concentration 0.005% to 0.05% by weight. Any appropriately cationic surfactant known in the art can be used in uric acid detecting electrode of the invention. Preferably, said cationic surfactant includes ammonium surfactants. Such cationic surfactants are preferably quaternary ammonium salts which have, as N-substituent, at least one C₈-C₂₂ alkyl radical and, as further substituents, lower unsubstituted or halogenated alkyl, benzyl or lower hydroxyalkyl radicals. The salts are preferably in the form of halides, methylsulfates or ethylsulfates. More preferably, such surfactant is selected from the group consisting of N-acetyl-N,N,N-trimethyl-ammonium bromide and alkyldimethylammonium halogenides. Most preferably, the concentration of said cationic surfactant ranges from 0.01% to 0.03% by weight.

Further information about surfactants may be found for example, in "McCutcheon's Detergents and Emulsifiers Annual", MC Publishing Corp. Ringwood, New Jersey, 1979, and Sisely and Wood, "Encyclopaedia of Surface Active Agents," Chemical Publishing Co., Inc. New York, 1980.

### Preparation of Electrode Strip

Another object of the invention is to provide a method for producing disposable non-enzymatic uric acid detecting electrode strip which comprises:
(a) coating a conducting film on one side of an electric insulating substrate and forming isolated and disconnected an anode and a cathode;
(b) coating an electric insulating film on a part of the conducting film, wherein one end of an uncovered anode of the conducting film is at least a reference electrode and the other end an anode connector, and one end of an uncovered cathode of the conducting film is at least a working electrode and the other end a cathode connector; and
(c) screen printing or pipetting a reaction film on a region containing at least the working electrode and the reference electrode so as to connect the working electrode and the reference electrode individually.

According to the invention, first, a conducting film is coated on one side of a flat substrate to form at least an anode and a cathode that are separately isolated from each other. After coating, the conducting film is dried at 35°C to 80°C.

According to the invention, secondly, an electric insulating film with a thickness of 0.6 mm or above is partially printed onto the conducting film. The uncovered parts of the conducting film form a cathode connector, an anode connector, a working electrode and a reference electrode. An area formed by the working electrode and the reference electrode in a circle or any other suitable shape is an area of reaction film.

According to the invention, thirdly, a reaction film is pipetting or screen printing on a reaction region so that the individual working electrode and the reference electrode can be connected. Said region can be a circle or any other suitable shape. Several conventional screen printing technologies can be used in the invention. Moreover, a new screen printing technology disclosed by one of the inventors of the present invention and contained in R.O.C. Patent Application Number 85,109,554 could also be applied in the production method of the present invention.

According to the invention, fourthly, the reaction film can be further dried at 40 to 80° C. A protection film is optionally coated on and around the area of the reaction film. The disposable non-enzymatic uric acid detecting electrode strip is therefore produced.

### Detection Equipment

Another further object is to provide an uric acid detecting equipment comprises a disposable non-enzymatic uric acid detecting electrode strip and a amperometric sensor directly analyzing uric acid in liquid sample,
wherein the electrode strip comprises:
(1) an electric insulating substrate;
(2) a conducting film coated on one side of said insulting substrate to form isolated and disconnected an anode and a cathode;
(3) an electric insulating film coated on a part of said conducting film, wherein one end of an uncovered anode of the conducting film forms at least a reference electrode and the other end an anode connector, and one end of an uncovered cathode of the conducting film forms at least a working electrode and the other end a cathode connector; and
(4) a reaction film comprising a carrier, a conductive mediator and a cationic surfactant with the concentration 0.005% to 0.05%, and being coated on a region containing at least said working electrode and said reference electrode so as to connect said working electrode and said reference electrode individually wherein said reaction film does not contain an enzyme; and wherein the amperometric sensor is connected to the anode connector and the cathode connector of the uric acid detecting electrode strip and comprises a voltage output equipment, a signal receiver and a display equipment; in which the voltage output equipment provides a voltage of 100 mV to 500 mV, preferably below 400 mV to the reaction film of the uric acid detecting electrode strip so as to oxidize the conductive mediator from reduction state to oxidation state after being reacted with uric acid of the sample; the signal receiver receives a current, voltage or resistance change generated during a redox reaction and transmits the change to the display equipment to display the concentration of uric acid in the sample.

According to the invention, the detecting conditions at a low operation voltage of below 400 mV and at a pH value from 5.0 to 10.0 avoid interference caused by other oxidizing components.

### Detection Method

Another object of the invention is to provide a method for detecting a concentration of uric acid in liquid, which comprises dropping the liquid on a non-enzymatic disposable uric acid detecting electrode strip of the invention, and controlling an amperometric sensor to be operated at a low operation voltage of below 400 mV and a pH value in the range from 7.0 to 10.0.

According to the invention, the detecting method of non-enzymatic electrode strip can proceed easily by using electrochemical detecting equipment. The reaction current caused by the redox reaction of uric acid can be detected in a uric acid detecting equipment by dropping a whole-blood sample on the reaction area of the non-enzymatic disposable uric acid detecting electrode strip of the present invention. Such an electrochemical reaction technology is commonly applied in electrochemical blood sugar monitor for detecting blood sugar. The method of directly detecting uric acid in whole-blood is novel and first disclosed in the present invention by using a redox electron mediator to transfer signals of the redox reaction of uric acid and by controlling the reaction at a pH value from 7.0 to 10.0 as well as a low operation voltage of below 400 mV to avoid the interference result from glucose and ascorbic acid in blood.

The present invention can be used in the detection of biomolecules such as uric acid in any liquid samples. The sample which may be tested in accordance with the invention may be any body fluid, preferably blood or a component of blood, more preferably whole blood. When taking whole-blood as a sample for detecting uric acid , there will be interference caused by other components, especially ascorbic acid in the blood The disposable detecting electrode strip and detecting equipment of the present invention can directly detect the uric acid in whole-blood for household use.

One important advantage of the present invention is to eliminate the use of any bio-active substances such as enzymes. So, the present invention simplifies the production process, reduces the production cost, increases the storage term, and releases the storage restriction of the detecting electrode strip.

### DETAILED DESCRIPTIONS OF PREFERRED EMBODIMENT OF THE INVENTION

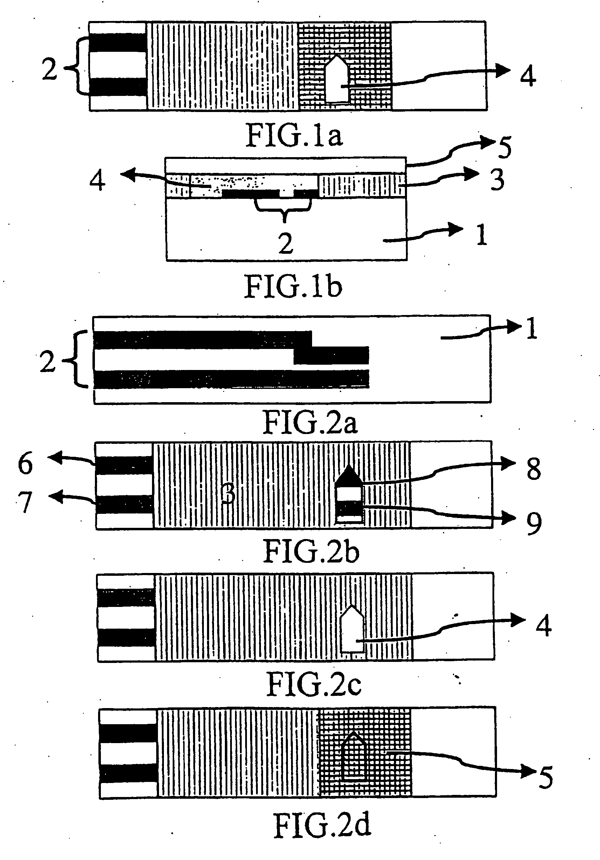

Figs. 1a and 1b are respectively a top view diagram and a front view diagram of an electrode strip of the present invention. The electrode strip structure comprises an electric insulating substrate 1, a conducting film 2 coated on the insulating substrate, an electric insulating film 3 coated on a part of the conducting film 2, and a reaction film 4 for reacting with a sample.

The electric insulating substrate 1 of the present invention adheres to the conducting film 2. The cathode of the conducting film 2 is partially covered by the electric insulating film 3 and two uncovered ends of the cathode are a working electrode 8 and a cathode connector 6 respectively. The working electrode 8 of the cathode is covered by the reaction film 4 and is used to detect an induced electric effect of samples during the electrochemical reaction of uric acid. The cathode connector 6 is used to connect with a amperometric sensor. The anode is also partially covered by the electric insulating film 3 and two uncovered ends of the anode are a reference electrode 9 and an anode connector 7 respectively. The reference electrode 9 of the anode is covered by the reaction film 4 and cooperates together with the working electrode of the cathode to detect the induced electric effect.

The preparation of the electrode strip is illustrated in Figures 2a, 2b, 2c and 2d. As shown in Figure 2a, a conducting film 2 is first coated such as by screen printing on one side of a flat substrate 1 to form at least an anode and a cathode which are separately isolated from each other.

As shown in Figure 2b, the uncovered parts of the conducting film form a cathode connector 6, an anode connector 7, a working electrode 8 and a reference electrode 9. An area formed by the working electrode 8 and the reference electrode 9 in a circle.

Further, as shown in Figures 2c and 2d, a conductive mediator are screen printing on the circle area of the reaction film 4 and a protection film 5 is optionally coated on and around the circle area of the reaction film. 4.

The following examples are exemplified to describe the present invention in detail but not to confine the present invention.

### Example 1 Detection of Biochemical Materials

The detection of uric acid was controlled at a pH value from 7.0 to 10.0 as well as a low operation voltage of below 400 mV to avoid the interference result from glucose and ascorbic acid in blood. It found that the vitamin C within the normal concentration would not affect the measurement of the uric acid. Thus, the biochemical materials can be accuracy determined.

### Example 2 The Detection of Uric Acid

On one flat side of polyvinyl chlorine (PVC) substrate, carbon ink was screen printed to form a conducting film 2 comprising a set of isolated and disconnected anode and cathode and then dried at 40 to 80° C. An electric insulating film 3 with a thickness of about 0.6 mm was subsequently screen printed on the conducting film 2 partially. The uncovered part of the conducting film formed a cathode connector 6, a anode connector 7, a working electrode 8 and a reference electrode 9. The circle area formed by working electrode 8 and reference electrode 9 was an area of reaction film 4.

Slurry materials comprising the following components and proportions were then pointed with pipet on the circle area of reaction film 4.

| | |
|---|---|
| Methyl cellulose | 0.01 % |
| CTAB (N-acetyl-N,N,N-tnmethyl-ammonium bromide) | 0.01 % |
| H₂O | 99.68 % |
| Potassium ferricyanide | 0.3 % |

After pointing said material on the reaction film 4, it was dried at 35 to 80° C. A protection film 5 was coated on and around the circle area of reaction film 4. The disposable non-enzymatic uric acid detecting electrode strip was produced through the production steps of the above.

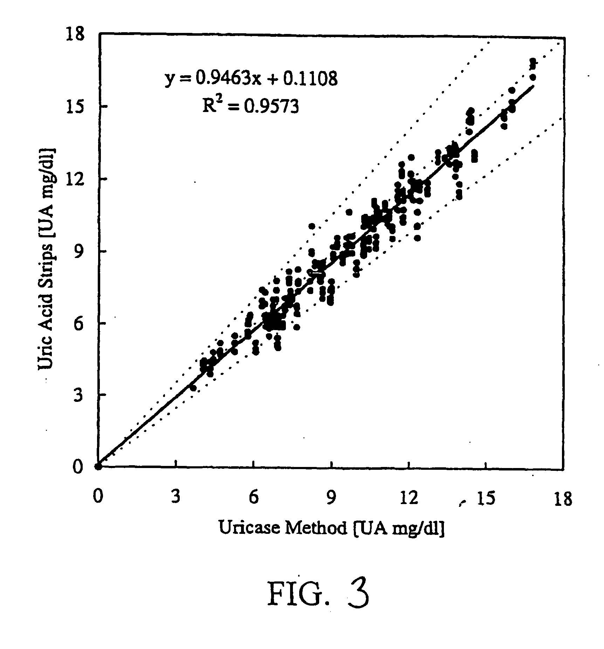

The uric acid in blood was detected for 20 seconds by the above-obtained disposable non-enzymatic uric acid detecting electrode strip of the present invention by taking whole-blood as a sample. The test results were the same as the uric acid concentration detected by any conventional method. Fig. 3 shows the comparative results of the uric acid concentration in serum by uricase method in combination with EPAC bio-analyzer.

The results show that the disposable non-enzymatic uric acid detecting electrode strip of the present invention can precisely detect the uric acid concentration in blood by taking whole-blood as a sample and does not require any pretreatment.

### Example 3 The Detection of Uric Acid

The steps of example 2 were repeated, except that the slurry materials were screen printed on the circle area of reaction film and the components and proportions of slurry materials were changed as follows:

| | |
|---|---|
| Methyl cellulose | 0.05 % |
| PEG, polyethylene glycol | 15.00 % |
| CTAB (N-acetyl-N,N,N-trimethyl-ammonium bromide) | 0.03 % |
| H₂O | 83.092 % |
| Potassium ferricyanide | 1.00 % |

The uric acid in blood was detected for 30 seconds by the above obtained disposable non-enzymatic uric acid detecting electrode strip of the present invention by taking whole-blood as a sample. The test results were the same as the uric acid concentration detected by uricase method in combination with EPAC bio-analyzer. The results show that the disposable non-enzymatic uric acid detecting electrode strip of the present invention can precisely detect the uric acid concentration in blood by taking whole-blood as a sample and does not require any pretreatment.

### Comparable Example 4 Detection of Uric Acid

The detection of the uric acid was respectively performed by the electrode strip of the invention and the electrode strip of USSN 09/295,400 that is filed by the Applicant on 21 April 1999. The results of five samples with different concentrations are shown as below table:

| Whole blood sample | | 1 | 2 | 3 | 4 | 5 | 6 |
|---|---|---|---|---|---|---|---|
| Reference Uricase Method | Average (mg/dl) | 3.55 | 5.11 | 7.35 | 9.10 | 10.20 | 11.85 |
| Electrode strip of the invention | Average (mg/dl) | 3.60 | 5.20 | 7.10 | 8.90 | 10.40 | 11.90 |
| | %CV | 3.80 | 4.50 | 3.40 | 3.40 | 5.40 | 2.00 |
| Electrode strip of USSN 09/295,400 | Average (mg/dl) | 13.50 | 5.60 | 6.80 | 8.90 | 10.50 | 12.00 |
| | %CV | 12.00 | 8.50 | 7.30 | 6.00 | 4.00 | 4.60 |

As shown in the table, the electrode strips of the invention and USSN 09/295,400 both have high accuracy. However, as shown in the values of CV%, the precision of the electrode strip of the invention is better than that of USSN 09/295,400. In addition, the electrode strip of the invention merely needs 1/10 of detection time than that of USSN 09/293,400.

## Claims

1. A disposable electrode article suitable for the non-enzymatic detection of uric acid said article comprising:
(1) an electric insulating substrate;
(2) a conducting film coated on one side of said insulting substrate to form an isolated and disconnected anode and an isolated and disconnected cathode;
(3) an electric insulating film coated on a part of said conducting film, wherein one portion of the conducting film anode forms an anode connector and another portion of the conducting film anode, which is uncoated by the insulating film, forms at least one reference electrode, and one portion of the conducting film cathode forms a cathode connector and another portion of the conducting film cathode, which is uncoated by the insulating film, forms at least one working electrode, and
(4) a reaction film comprising a carrier, a conductive mediator and a cationic surfactant, the surfactant being present in a concentration of 0.005% to 0.05% by weight, said reaction film being coated on a region containing at least said working electrode and said reference electrode so as to permit electrical connection of said working electrode and said reference electrode via said reaction film.

2. The electrode article of claim 1, wherein said electric insulating substrate has a flat surface.

3. The electrode article of claim 1 or 2 wherein said electric insulating substrate is thermally-resistant at temperatures of more than 40°C, preferably 40-80°C.

4. The electrode article of any one of the preceding claims wherein said electric insulating substrate comprises polyvinyl chloride (PVC), fiber glass (FR-4), polyester sulphone bakelite plate, polyethylene terephthalate (PET) plate, polycarbonate (PC) plate, glass plate or ceramics plate (CEM-1).

5. The electrode article of any one of the preceding claims, wherein said reaction film when contacted with a test sample is capable of undergoing an electrochemical reaction.

6. The electrode article of any one of the preceding claims, wherein said reaction film is further covered with a protection film.

7. The electrode article of any one of the preceding claims, wherein said carrier comprises microcrystalline cellulose, carboxymethyl cellulose or methyl cellulose.

8. The electrode article of claim 7, wherein said carrier is microcrystalline cellulose having a size below 100 µm.

9. The electrode article of claim 7 or 8, wherein the amount of said carrier cellulose ranges from 0.01% to 0.1 % by weight of the reaction film.

10. The electrode article of any one of the preceding claims, wherein said carrier optionally comprises a polymer which is polyvinyl alcohol (PVA), polyvinyl pyrrolidone (PVP), polyethylene glycol (PEG), or gelatin.

11. The electrode article of claim 10, wherein the amount of said polymer is from 0 to 15% by weight of the reaction film.

12. The electrode article of any one of the preceding claims, wherein said conductive mediator is K₃Fe(CN)₆

13. The electrode article of any one of the preceding claims, wherein the amount of said conductive mediator is from 0.3% to 5% by weight of the reaction film.

14. The electrode article of any one of the preceding claims wherein the cationic surfactant is N-acetyl-N,N,N-trimethyl-ammonium bromide.

15. The electrode article of any one of the preceding claims, wherein the surfactant is present in the reaction film in an amount of from 0.01% to 0.03% by weight.

16. A process for producing the electrode article as claimed in any one of the preceding claims, which process comprises:
(a) coating a conducting film on one side of an electric insulating substrate and forming an isolated and disconnected anode and an isolated and disconnected cathode;
(b) coating an electric insulating film on a part of the conducting film, wherein one portion of the conducting film anode forms an anode connector and another portion of the conducting film anode, which is uncoated by the insulating film, forms a reference electrode and one portion of the conducting film cathode forms a cathode connector and another portion of the conducting film cathode, which is uncoated by the insulating film, forms a working electrode; and .
(c) forming a reaction film on a region containing at least the working electrode and the reference electrode so as to permit electrical connection of the working electrode and the reference electrode.

17. The process of claim 16 wherein the reaction film is formed by screen printing or pipetting the film on said region.

18. Apparatus suitable for the non-enzymatic detection of uric acid in a liquid sample, said apparatus comprising an electrode article and an amperometric sensor; wherein the electrode article comprises:
(1) an electric insulating substrate;
(2) a conducting film coated on one side of said insulting substrate to form an isolated and disconnected anode and an isolated and disconnected cathode;
(3) an electric insulating film coated on a part of said conducting film, wherein one portion of the conducting film anode forms an anode connector and another portion of the conducting film anode, which is uncoated by the insulating film, forms at least one reference electrode, and one portion of the conducting film cathode forms a cathode connector and another portion of the conducting film cathode, which is uncoated by the insulating film, forms at least one working electrode, and
(4) a reaction film comprising a carrier, a conductive mediator and a cationic surfactant, the surfactant being present in a concentration of 0.005% to 0.05% by weight, said reaction film being coated on a region containing at least said working electrode and said reference electrode so as to permit electrical connection of said working electrode and said reference electrode via said reaction film; and
wherein the amperometric sensor is connected to the anode connector and the cathode connector of the electrode article, said sensor comprising a voltage output equipment, a signal receiver and a display equipment; such that the voltage output equipment is capable of providing a voltage of 100 mV to 500 mV to the reaction film of the electrode article so as to oxidize the conductive mediator from reduction state to oxidation state after being reacted with uric acid of the sample; the signal receiver is capable of receiving a current, voltage or resistance change generated during a redox reaction and transmitting the change to the display equipment to display the concentration of uric acid in the sample.

19. The apparatus of claim 18 wherein said detecting electrode article is disposable.

20. The equipment of claim 18 or 19 adapted to measure a liquid sample which is blood.

21. A method for detecting the concentration of uric acid in a liquid, which comprises dropping a sample of the liquid on a disposable uric acid detecting electrode article of claim 12, and controlling the amperometric sensor to be operated at a voltage of 100 mV to 500 mV and a pH value of from 7.0 to 10.0.

## Patentansprüche

1. Einweg-Elektrodengegenstand, der sich für den nicht-enzymatischen Nachweis von Harnsäure eignet, wobei der Gegenstand folgendes umfasst:
(1) ein elektrisch isolierendes Substrat;
(2) einen leitfähigen Film, der auf eine Seite des isolierenden Substrats aufgebracht ist, um eine isolierte und abgetrennte Anode und eine isolierte und abgetrennte Kathode zu bilden;
(3) einen elektrisch isolierenden Film, der auf einen Teil des leitfähigen Films aufgebracht ist, wobei ein Bereich der leitfähigen Filmanode ein Anoden-Verbindungselement bildet und ein weiterer Bereich der leitfähigen Filmanode, der nicht mit dem isolierenden Film beschichtet ist, mindestens eine Referenzelektrode bildet, und ein Bereich der leitfähigen Filmkathode ein Kathoden-Verbindungselement bildet und ein weiterer Bereich der leitfähigen Filmkathode, der nicht mit dem isolierenden Film beschichtet ist, mindestens eine Arbeitselektrode bildet, und
(4) ein Reaktionsfilm, der einen Träger, einen leitfähigen Vermittler und ein kationisches oberflächenaktives Mittel umfasst, wobei das oberflächenaktive Mittel in einer Konzentration von 0,005 bis 0,05 Gew.-% vorhanden ist, wobei der Reaktionsfilm auf einem Bereich, der mindestens die Arbeitselektrode und die Referenzelektrode enthält, aufgebracht ist, um eine elektrische Verbindung der Arbeitselektrode und der Referenzelektrode über diesen Reaktionsfilm zu ermöglichen.

2. Elektrodengegenstand nach Anspruch 1, wobei das elektrisch isolierende Substrat eine flache Oberfläche aufweist.

3. Elektrodengegenstand nach Anspruch 1 oder 2, wobei das elektrisch isolierende Substrat bei Temperaturen von mehr als 40 °C und vorzugsweise von 40-80 °C thermisch beständig ist.

4. Elektrodengegenstand nach einem der vorstehenden Ansprüche, wobei das elektrisch isolierende Substrat Polyvinylchlorid (PVC), Glasfaserstoff (FR-4), Polyestersulfon, eine Bakelit-Platte, eine Polyethylenterephthalat (PET)-Platte, eine Polycarbonat (PC)-Platte, eine Glasplatte oder eine Keramikplatte (CEM-1) umfasst.

5. Elektrodengegenstand nach einem der vorstehenden Ansprüche, wobei der Reaktionsfilm bei Kontakt mit einer Testprobe dazu befähigt ist, eine elektrochemische Reaktion einzugehen.

6. Elektrodengegenstand nach einem der vorstehenden Ansprüche, wobei der Reaktionsfilm ferner mit einem Schutzfilm bedeckt ist.

7. Elektrodengegenstand nach einem der vorstehenden Ansprüche, wobei der Träger mikrokristalline Cellulose, Carboxymethylcellulose oder Methylcellulose umfasst.

8. Elektrodengegenstand nach Anspruch 7, wobei der Träger mikrokristalline Cellulose mit einer Größe unter 100 µm umfasst.

9. Elektrodengegenstand nach Anspruch 7 oder 8, wobei die Menge der Trägercellulose im Bereich von 0,01 bis 0,1 Gew.-% des Reaktionsfilms liegt.

10. Elektrodengegenstand nach einem der vorstehenden Ansprüche, wobei der Träger gegebenenfalls ein Polymeres umfasst, bei dem es sich um Polyvinylalkohol (PVA), Polyvinylpyrrolidon (PVP), Polyethylenglycol (PEG) oder Gelatine handelt.

11. Elektrodengegenstand nach Anspruch 10, wobei die Menge des Polymeren 0 bis 15 Gew.-% des Reaktionsfilms beträgt.

12. Elektrodengegenstand nach einem der vorstehenden Ansprüche, wobei es sich beim leitfähigen Vermittler um K₃Fe(CN)₆ handelt.

13. Elektrodengegenstand nach einem der vorstehenden Ansprüche, wobei die Menge des leitfähigen Vermittlers 0,3 bis 5 Gew.-% des Reaktionsfilm beträgt.

14. Elektrodengegenstand nach einem der vorstehenden Ansprüche, wobei es sich beim kationischen oberflächenaktiven Mittel um N-Acetyl-N,N,N-trimethylammoniumbromid handelt.

15. Elektrodengegenstand nach einem der vorstehenden Ansprüche, wobei das oberflächenaktive Mittel im Reaktionsfilm in einer Menge von 0,01 bis 0,03 Gew.-% vorhanden ist.

16. Verfahren zur Herstellung des Elektrodengegenstands nach einem der vorstehenden Ansprüche, wobei das Verfahren folgendes umfasst:
(a) das Aufbringen eines leitfähigen Films auf eine Seite eines elektrisch isolierenden Substrats und das Bilden einer isolierten und abgetrennten Anode und einer isolierten und abgetrennten Kathode;
(b) das Aufbringen eines elektrisch isolierenden Films auf einen Teil des leitfähigen Films, wobei ein Bereich der leitfähigen Filmanode ein Anoden-Verbindungselement bildet und ein weiterer Bereich der leitfähigen Filmanode, der nicht mit dem isolierenden Film beschichtet ist, eine Referenzelektrode bildet, und ein Bereich der leitfähigen Filmkathode ein Kathoden-Verbindungselement bildet und ein weiterer Bereich der leitfähigen Filmkathode, der nicht mit dem isolierenden Film beschichtet ist, eine Arbeitselektrode bildet, und
(c) das Bilden eines Reaktionsfilms auf einen Bereich, der mindestens die Arbeitselektrode und die Referenzelektrode enthält, um eine elektrische Verbindung der Arbeitselektrode und der Referenzelektrode zu ermöglichen.

17. Verfahren nach Anspruch 16, wobei der Reaktionsfilm durch Siebdruck oder Pipettieren des Films auf diesen Bereich gebildet wird.

18. Vorrichtung, die sich zum nicht-enzymatischen Nachweis von Harnsäure in einer flüssigen Probe eignet, wobei die Vorrichtung einen Elektrodengegenstand und einen amperometrischen Sensor umfasst, wobei der Elektrodengegenstand folgendes umfasst:
(1) ein elektrisch isolierendes Substrat;
(2) einen leitfähigen Film, der auf eine Seite des isolierenden Substrats aufgebracht ist, um eine isolierte und abgetrennte Anode und eine isolierte und abgetrennte Kathode zu bilden;
(3) einen elektrisch isolierenden Film, der auf einen Teil des leitfähigen Films aufgebracht ist, wobei ein Bereich der leitfähigen Filmanode ein Anoden-Verbindungselement bildet und ein weiterer Bereich der leitfähigen Filmanode, der nicht mit dem isolierenden Film beschichtet ist, mindestens eine Referenzelektrode bildet, und ein Bereich der leitfähigen Filmkathode ein Kathoden-Verbindungselement bildet und ein weiterer Bereich der leitfähigen Filmkathode, der nicht mit dem isolierenden Film beschichtet ist, mindestens eine Arbeitselektrode bildet, und
(4) ein Reaktionsfilm, der einen Träger, einen leitfähigen Vermittler und ein kationisches oberflächenaktives Mittel umfasst, wobei das oberflächenaktive Mittel in einer Konzentration von 0,005 bis 0,05 Gew.-% vorhanden ist, wobei der Reaktionsfilm auf einem Bereich, der mindestens die Arbeitselektrode und die Referenzelektrode enthält, aufgebracht ist, um eine elektrische Verbindung der Arbeitselektrode und der Referenzelektrode über diesen Reaktionsfilm zu ermöglichen; und
wobei der amperometrische Sensor mit dem Anoden-Verbindungselement und dem Kathoden-Verbindungselement des Elektrodengegenstands verbunden ist, wobei der Sensor eine Spannungsausgabeeinrichtung, einen Signalempfänger und eine Anzeigeeinrichtung umfasst; so dass die Spannungsausgabeeinrichtung zum Anlegen einer Spannung von 100 mV bis 500 mV an den Reaktionsfilm des Eletrodengegenstands befähigt ist, um den leitfähigen Vermittler vom Reduktionszustand in den Oxidationszustand zu oxidieren, nachdem er mit Harnsäure der Probe reagiert hat; wobei der Signalempfänger zum Empfang einer Strom-, Spannungs- oder Widerstandsänderung, die während einer Redoxreaktion erfolgt, und zur Übertragung der Veränderung an die Anzeigeeinrichtung befähigt ist, um die Harnsäurekonzentration der Probe anzuzeigen.

19. Vorrichtung nach Anspruch 18, wobei es sich beim Nachweis-Elektrodengegenstand um einen Einweggegenstand handelt.

20. Vorrichtung nach Anspruch 18 oder 19, der zur Messung einer flüssigen Probe, bei der es sich um Blut handelt, geeignet ist.

21. Verfahren zum Nachweisen der Konzentration an Harnsäure in einer Flüssigkeit, das das Auftropfen einer Probe der Flüssigkeit auf einen Einweg-Harnsäurenachweis-Elektrodengegenstand von Anspruch 12 unter Steuern des amperometrischen Sensors zum Betrieb einer Spannung von 100 mV bis 500 mV und einem pH-Wert von 7,0 bis 10,0 umfasst.

## Revendications

1. Élément d'électrode jetable convenant pour la détection non enzymatique de l'acide urique, ledit élément comprenant :
(1) un substrat isolant électrique ;
(2) un film conducteur revêtu sur un côté dudit substrat isolant pour former une anode isolée et déconnectée et une cathode isolée et déconnectée ;
(3) un film isolant électrique revêtu sur une partie dudit film conducteur, dans lequel une portion de l'anode à film conducteur forme un connecteur d'anode et une autre portion de l'anode à film conducteur, qui n'est pas revêtue du film isolant, forme au moins une électrode de référence, et une portion de la cathode à film conducteur forme un connecteur de cathode et une autre portion de la cathode à film conducteur, qui n'est pas revêtue du film isolant, forme au moins une électrode de travail, et
(4) un film réactif comprenant un support, un médiateur conducteur et un agent tensioactif cationique, l'agent tensioactif étant présent en une concentration de 0,005 % à 0,05 % en poids, ledit film réactif étant revêtu sur une région contenant au moins ladite électrode de travail et ladite électrode de référence afin de permettre une connexion électrique de ladite électrode de travail et de ladite électrode de référence par l'intermédiaire dudit film réactif.

2. Élément d'électrode selon la revendication 1, dans lequel ledit substrat isolant électrique a une surface plate.

3. Élément d'électrode selon la revendication 1 ou 2, dans lequel ledit substrat isolant électrique est thermorésistant à des températures supérieures à 40°C, de préférence à 40 - 80°C.

4. Élément d'électrode selon l'une quelconque des revendications précédentes, dans lequel ledit substrat isolant électrique comprend un chlorure de polyvinyle (PVC), une fibre de verre (FR-4), un polyester sulfoné, une plaque de bakélite, une plaque de téréphtalate de polyéthylène (PET), une plaque de polycarbonate (PC), une plaque de verre ou une plaque de céramique (CEM-1).

5. Élément d'électrode selon l'une quelconque des revendications précédentes, dans lequel ledit film réactif, lorsqu'il est mis en contact avec un échantillon d'essai, est capable de subir une réaction électrochimique.

6. Élément d'électrode selon l'une quelconque des revendications précédentes, dans lequel ledit film réactif est en outre revêtu d'un film de protection.

7. Élément d'électrode selon l'une quelconque des revendications précédentes, dans lequel ledit support comprend de la cellulose microcristalline, de la carboxyméthylcellulose ou de la méthylcellulose.

8. Élément d'électrode selon la revendication 7, dans lequel ledit support est de la cellulose microcristalline ayant une taille inférieure à 100 µm.

9. Élément d'électrode selon la revendication 7 ou 8, dans lequel la quantité dudit support de cellulose est dans la gamme de 0,01 % à 0,1 % en poids du film réactif.

10. Élément d'électrode selon l'une quelconque des revendications précédentes, dans lequel ledit support comprend facultativement un polymère qui est un alcool polyvinylique (PVA), une polyvinylpyrrolidone (PVP), un polyéthylèneglycol (PEG) ou de la gélatine.

11. Élément d'électrode selon la revendication 10, dans lequel la quantité dudit polymère est de 0 à 15 % en poids du film réactif.

12. Élément d'électrode selon l'une quelconque des revendications précédentes, dans lequel le médiateur conducteur est K₃Fe(CN)_{6.}

13. Élément d'électrode selon l'une quelconque des revendications précédentes, dans lequel la quantité dudit médiateur conducteur est dans la gamme de 0,3 % à 5 % en poids du film réactif.

14. Élément d'électrode selon l'une quelconque des revendications précédentes, dans lequel l'agent tensioactif est le bromure de N-acétyl-N,N,N-triméthyl-ammonium.

15. Élément d'électrode selon l'une quelconque des revendications précédentes, dans lequel le tensioactif est présent dans le film réactif en une quantité de 0,01 % à 0,03 % en poids.

16. Procédé pour produire l'élément d'électrode tel que revendiqué dans l'une quelconque des revendications précédentes, lequel procédé comprend :
(a) le revêtement d'un film conducteur sur un côté d'un substrat isolant électrique et la formation d'une anode isolée et déconnectée et d'une cathode isolée et déconnectée ;
(b) le revêtement d'un film isolant électrique sur une partie du film conducteur, dans lequel une portion de l'anode à film conducteur forme un connecteur d'anode et une autre portion de l'anode à film conducteur, qui n'est pas revêtue du film isolant, forme une électrode de référence et une portion de la cathode à film conducteur forme un connecteur de cathode et une autre portion de la cathode à film conducteur, qui n'est pas revêtue du film isolant, forme une électrode de travail ; et
(c) la formation d'un film réactif sur une région contenant au moins l'électrode de travail et l'électrode de référence afin de permettre une connexion électrique de l'électrode de travail et de l'électrode de référence.

17. Procédé selon la revendication 16, dans lequel le film réactif est formé par sérigraphie ou pipetage du film sur ladite région.

18. Appareil convenant pour la détection non enzymatique d'acide urique dans un échantillon liquide, ledit appareil comprenant un élément d'électrode et un capteur ampérométrique ; dans lequel l'élément d'électrode comprend :
(1) un substrat isolant électrique ;
(2) un film conducteur revêtu sur un côté dudit substrat isolant pour former une anode isolée et déconnectée et une cathode isolée et déconnectée ;
(3) un film isolant électrique revêtu sur une partie dudit film conducteur, dans lequel une portion de l'anode à film conducteur forme un connecteur d'anode et une autre portion de l'anode à film conducteur, qui n'est pas revêtue du film isolant, forme au moins une électrode de référence, et une portion de la cathode à film conducteur forme un connecteur de cathode et une autre portion de la cathode à film conducteur, qui n'est pas revêtue du film isolant, forme au moins une électrode de travail, et
(4) un film réactif comprenant un support, un médiateur conducteur et un agent tensioactif cationique, l'agent tensioactif étant présent en une concentration de 0,005 % à 0,05 % en poids, ledit film réactif étant revêtu sur une région contenant au moins ladite électrode de travail et ladite électrode de référence afin de permettre une connexion électrique de ladite électrode de travail et de ladite électrode de référence par l'intermédiaire dudit film réactif, et
dans lequel le capteur ampérométrique est connecté au connecteur d'anode et au connecteur de cathode de l'élément d'électrode, ledit capteur comprenant un équipement de tension de sortie, un récepteur de signal et un équipement d'affichage ; de sorte que l'équipement de tension de sortie est capable de fournir une tension de 100 mV à 500 mV au film réactif de l'élément d'électrode afin d'oxyder le médiateur conducteur qui passe de l'état de réduction à l'état d'oxydation après l'avoir fait réagir avec l'acide urique de l'échantillon ; le récepteur de signal est capable de recevoir une modification de courant, de tension ou de résistance générée pendant une réaction redox et de transmettre la modification à l'équipement d'affichage pour afficher la concentration en acide urique de l'échantillon.

19. Appareil selon la revendication 18, dans lequel ledit élément d'électrode à détection est jetable.

20. Dispositif selon la revendication 18 ou 19 conçu pour mesurer un échantillon liquide qui est le sang.

21. Procédé pour détecter la concentration d'acide urique dans un liquide,
qui comprend la tombée goutte à goutte d'un échantillon de liquide sur un élément d'électrode jetable détectant l'acide urique selon la revendication 12, et le contrôle du capteur ampérométrique devant fonctionner sous une tension de 100 mV à 500 mV et à une valeur de pH de 7,0 à 10,0.
